Europäisches Patentamt

(19) European Patent Office

Office européen des brevets

(11) Publication number: **0 215 623**
**A2**

(12) # EUROPEAN PATENT APPLICATION

(21) Application number: 86306905.0

(22) Date of filing: 08.09.86

(51) Int. Cl.⁴: **C 12 P 19/04**

(30) Priority: 09.09.85 US 773766

(43) Date of publication of application:
25.03.87 Bulletin 87/13

(84) Designated Contracting States:
**BE DE FR GB IT NL**

(71) Applicant: **CELANESE CORPORATION**
**1211 Avenue of the Americas**
**New York New York 10036 (US)**

(72) Inventor: **Towle, Gordon**
**97 W. Main Street**
**Niantic Connecticut (US)**

**Stirling, Davis I.**
**4 Lois Place**
**Eanwood New Jersey (US)**

(74) Representative: **De Minvielle-Devaux, Ian Benedict Peter**
**et al**
**CARPMAELS & RANSFORD 43, Bloomsbury Square**
**London WC1A 2RA (GB)**

(54) **Production of novel heteropolysaccharides.**

㊼ This invention provides a process which involves aerobically cultivating a strain of Methylophilus viscogenes under growth conditions to produce an accumulated quantity of a novel type of exopolysaccharide, such as heteropolysaccharide Poly 54, and treating the exopolysaccharide under alkaline conditions to provide a deacetylated heteropolysaccharide which imparts enhanced pseudoplastic and thixotropic properties to aqueous solutions.

EP 0 215 623 A2

## Description

### BACKGROUND OF THE INVENTION

In the last decade the production of single-cell protein(SCP) from $C_1$-compounds has been studied extensively by academic and industrial laboratories. Enterprises such as Mitsubishi Gas Chemical Co., Hoechst, and I.C.I. have implemented pilot plant studies, but to date only I.C.I. is operating a full scale SCP plant. "Pruteen" is the registered trademark of the I.C.I. product and is made by the fermentation of the obligate methylotrophic bacterium. Methylophilus methylotrophus, which is eventually separated and dried to a powder or granules. Methylophilus methylotrophus is an obligate methylotroph which uses the ribulose monophosphate pathway(RMP) cycle of formaldehyde fixation. Methylophilus methylotrophus Strain AS-1 is a gram negative, nonpigmented rod with a single polar flagellum.

Although methanol and other $C_1$-compounds generally are viewed as substrates for the production of single cell protein, the factors that qualify $C_1$-compounds for SCP manufacture also recommend them as a potential feedstock for the production of accumulated extracellular metabolites such as exopolysaccharides. A number of microbial processes for the conversion of methanol to value-added products have been described but generally these are low-volume/high-priced compounds such as aminoacids. Thus, J. Bolbot and C. Anthony in Proc. Soc. Gen. Microbial, 5, 43(1978) found that a pyruvate dehydrogenase lacking mutant of Pseudomonas AMI could accumulate the aminoacids alanine and valine during growth on methanol. Y. Tani et al in Agric. Biol. Chem., 42, 2275(1978) have described the production of up to 5.2 grams per liter of L-serine from methanol employing as Arthrobacter globiformis strain. Up to the present time there has not been any report of $C_1$-compound bioconversion to a commodity type of bulk chemical.

Accordingly, it is an object of this invention to provide a fermentation process for the bioconversion of a growth carbon source to an accumulated quantity of extracellular metabolite.

It is another object of this invention to provide a process for the bio-oxidation of a $C_1$-compound to an accumulated quantity of an exopolysaccharide, and for the chemical modification of the exopolysaccharide to a novel heteropolysaccharide.

It is a further object of this invention to provide a novel heteropolysaccharide which exhibits properties suitable for imparting pseudoplastic and thixotropic properties to aqueous solutions.

Other objects and advantages of the present invention shall become apparent from the accompanying description and examples.

### DESCRIPTION OF THE INVENTION

One or more objects of the present invention are accomplished by the provision of a fermentation process which comprises aerobically cultivating a Methylophilus viscogenes strain in an aqueous nutrient medium containing a growth carbon source (e.g., a $C_1$-compound or fructose) to produce an accumulated quantity of extracellular heteropolysaccharide in the fermentation medium, separating the culture microorganism from the fermentation medium to provide a cell-free aqueous solution of heteropolysaccharide, and adjusting the pH of the aqueous solution into the alkaline range to deacetylate the heteropolysaccharide.

The deacetylated heteropolysaccharide product of the process is a novel composition of matter.

The whole cells can be removed from the fermentation broth by conventional means such as centrifugation, ultrafiltration or heat sterilization, and then the cell-free aqueous solution of exopolysaccharide metabolite can be subjected to the subsequent processing procedures for deacetylation and product recovery.

The term "$C_1$-compound" as employed herein refers to an organic compound which does not contain any carbon-carbon bonds, such as methanol, formaldehyde, formate, formamide, carbon monoxide, dimethyl ether, methylamine, dimethylamine, trimethylamine and trimethylamine N-oxide.

The term "exopolysaccharide" as employed herein refers to a polysaccharide which accumulates as an extracellular metabolite in a fermentation medium, as exemplified by xanthan gum, Polysaccharide S-60 (U.S. 4,326,052) and heteropolysaccharide Poly 54.

The term "heteropolysaccharide" as employed herein refers to a polysaccharide which is composed of at least two different kinds of monosaccharide units, such as a mannose and galactose.

In another embodiment this invention provides a fermentation process which comprises aerobically cultivating Methylophilus viscogenes strain ATCC 39893 or a variant thereof in a nutrient medium containing methanol as a growth carbon source to produce an accumulated quantity of extracellular heteropolysaccharide Poly 54 in the fermentation medium, and reacting the heteropolysaccharide Poly 54 under alkaline conditions to produce deacetylated heteropolysaccharide Poly 54.

An alternative means of producing a deacetylated heteropolysaccharide product in accordance with the present invention is to recover the extracellular heteropolysaccharide intermediate (e.g., heteropolysaccharide Poly 54) as a solid material, and then redissolve the recovered solid material in an aqueous medium where it can be modified by treatment under alkaline conditions to yield the desired deacetylated heteropolysaccharide product.

The alkaline treatment to modify the exopolysaccharide intermediate can be accomplished by adjusting the pH of the aqueous solution of the exopolysaccharide intermediate to a pH value between about 7-13, preferably to a pH between about 8-12 with an alkaline reagent at a temperature between about 20°-100°C for a treatment period of sufficient duration to effect hydrolytic modification of the exopolysaccharide. A typical reaction time is between about 0.1-2 hours. The alkaline reagent can be ammonia or an organic amine, or a basic inorganic compound such as sodium hydroxide, potassium carbonate, and the like.

A present invention product such as deacetylated heteropolysaccharide Poly 54 can be recovered as a solid product from an aqueous medium by conventional means, such as by dilution of the aqueous medium with a water-miscible organic solvent to precipitate the product. Illustrative of suitable organic solvent diluents are methanol, ethanol, propanol, acetone, tetrahydrofuran, and the like.

The deacetylated heteropolysaccharide also can be precipitated by treatment of the aqueous solution with a calcium salt.

The deacetylated heteropolysaccharide product contains carboxylic acid groups, and can exist either as a metal salt or in the free acid form.

In a further embodiment this invention provides a fermentation process which comprises aerobically cultivating a Methylophilus viscogenes strain in an aqueous nutrient medium containing a growth carbon source to produce an accumulated quantity of extracellular heteropolysaccharide in the aqueous medium, diluting the aqueous medium with a water-miscible organic solvent to precipitate the heteropolysaccharide to form an aqueous slurry medium, treating the aqueous slurry medium with an alkaline reagent to produce an aqueous slurry of deacetylated heteropolysaccharide.

This process embodiment has the advantage of producing a deacetylated heteropolysaccharide which in its recovered crude form is less color-contaminated and of higher purity.

Crude deacetylated heteropolysaccharide in precipitate form can be redissolved in water, and the aqueous solution then subjected to dialysis and lyophilization to provide a purified product.

In another embodiment for producing a deacetylated heteropolysaccharide product in accordance with the present invention, the extracellular heteropolysaccharide intermediate such as Poly 54 as recovered as a solid material, and then the solid material is treated with an alkaline solution (e.g., an aqueous alkanol caustic solution) to effect diacetylation of the said solid material. This method of heterogeneous phase deacetylation has the added advantages of ease of operation and less induced degradation of the heteropolysaccharide substrates.

The novel deacetylated heteropolysaccharide Poly 54 of the present invention has an average molecular weight in the range between about 800,000 and 1,500,000.

Deacetylated heteropolysaccharide Poly 54 nominally exhibits a 0.5% viscosity of at least about 1000 cps at 50 RPM, e.g., about 1050 cps at 50 RPM, as measured with Brookfield Spindle No. 4 at 25°C. An aqueous solution of deacetylated heteropolysaccharide Poly 54 is colorless, transparent and exhibits pseudoplastic and thixotropic viscosity properties. The aqueous solution viscosity properties are stable at temperatures up to 130°C, and at pH values up to about 12.

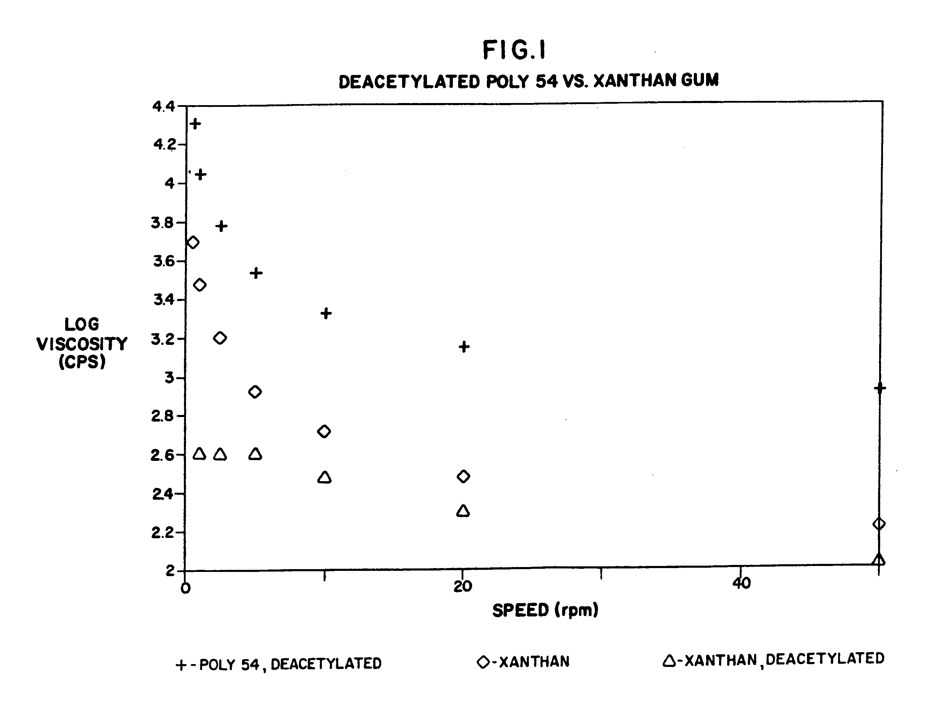

Under low shear rate conditions, deacetylated heteropolysaccharide Poly 54, exhibits an apparent viscosity which typically is about 10-30 times that of commercial xanthan gum. The comparative viscosity properties of deacetylated heteropolysaccharide Poly 54 and commercial xanthan (Kelzan) gum under shear conditions are illustrated in Figure 1.

Other physicochemical and structural features of deacetylated heteropolysaccharide Poly 54 are as follows:

## A. Constituent Sugars(mole %)

| | |
|---|---|
| glucose | 50 |
| galactose | 30 |
| mannose | 10 |
| glucuronic acid | 10 |

Deacetylated heteropolysaccharide Poly 54 contains no pyruvate or protein, and it contains less than about one acetyl group per four monosaccharide units. Usually the alkaline treatment removes all of the acetyl groups originally present in heteropolysaccharide Poly 54. Partially deacetylated Poly 54 also can be produced.

3

B. <u>Elemental Analysis (%)*</u>

|   |   |
|---|---|
| C | 38.22 |
| H | 5.57 |
| O | 40.41 |
| N | 1.58 |

*Ash corrected

C. Melting Point
No clear melting point, and decomposes above a temperature of about 150°C.

D. Infrared Spectrum
Band at 1740 $cm^{-1}$ which may indicate aliphatic ester groups.
Bands at 1650 $cm^{-1}$ and 1540 $cm^{-1}$ which may indicate amide groups.

E. Ultraviolet Absorption Spectrum
Broad max peak at 254 mu.

F. Solubility Properties
Soluble in water, but insoluble in all common organic solvents.

G. Specific Rotation
No measurable specific rotation.

Novel Species of Facultative Methylotrophic Bacterium
The present invention embodiments are accomplished by the utilization of novel strains of bacteria having identifying characteristics comprising:
(a) aerobic, gram-negative, rod-shaped, motile and polarly flagellated cells;
(b) methylotroph capable of assimilating methanol via the ribulose monophosphate pathway;
(c) capable of growth on fructose; and
(d) optimal growth rate at a cultivation medium temperature of 35°-40°C, and
In a particular embodiment this invention employs a bacterial culture having the identifying characteristics of strain ATCC 39893, said culture being capable of aerobic bioconversion of methanol or other $C_1$-compound to an extracellular accumulation of heteropolysaccharide.
Subcultures of accession Number ATCC 39893 strain can be obtained upon request from the permanent microorganism collection of the American Type Culture Collection, 12301 Parklawn Drive, Rockville, Maryland 20852.
The bacterial strains having the identifying characteristics of strain ATCC 39893 are not members of any of the known methylotrophic species such as <u>Methylophilus</u> <u>methylotrophus</u>. For purposes of taxonomic identification, the name <u>Methylophilus</u> <u>viscogenes</u> has been assigned to the new facultative methylotroph species which includes bacterial strains having the identifying characteristics of strain ATCC 39893.
The term "methylotroph" as employed herein refers to a microorganism which is capable of growing non-autotrophically on carbon compounds having one or more carbon atoms but no carbon-carbon bonds. "Autotrophic" refers to growth on a carbon dioxide substrate.
The term "facultative methylotroph" as employed herein refers to a methylotroph which is capable of growth on one or more heterotrophic substrates, e.g., fructose.
The term "ribulose monophosphate pathway" (RMP) as employed herein refers to the biochemical cycle in which three molecules of formaldehyde are condensed to produce either one molecule of pyruvate or one molecule of dihydroxyacetone phosphate.
Biochemical literature relating to elucidation of the ribulose monophosphate pathway and its variations include Biochem. J., 144, 465(1974) by J. Strøm et al; Sci. Prog., 62, 167(1975) by C. Anthony; and Biochem. J., 148, 513(1975) by Colby et al.
The ribulose monophosphate pathway involves enzymes which include G-phosphogluconate dehyrase/phospho-2-keto-3-digluconate aldolase, fructose diphosphate aldolase; glucose-6-phosphate dehydrogenase; 3-hexulose ohosphate synthase; phosphofructokinase; phosphoglucoisomerase; phospho-3-hexulose isomerase; phosphoriboisomerase; ribulose-5-phosphate 3-epimerase; transaldolase; transketolase; sedoheptulose diphosphate aldolase; and sedoheptulase-1,7-diphosphatase.

The RMP cycle effectively condenses 3 molecules of formaldehyde with 3 molecules of ribulose 5-phosphate to form 3 molecules of fructose 6-phosphate, which in turn regenerates 3 molecules of ribulose 5-phosphate via the cycle with the overall production of one molecule of dihydroxyacetone phosphate or pyruvic acid. These metabolites function as substrates for cellular biosynthesis.

The two key enzymes of the cycle are envisioned to be hexulase phosphate synthase and hexulase phosphate isomerase, which are the enzymes responsible for condensing formaldehyde and ribulose 5-phosphate and converting the product to fructose 6-phosphate. These are two recognized variants of the RMP cycle, i.e., the fructose bisphosphate variant which gives rise to dihydroxyacetone phosphate, and the Entner-Doudoroff variant which produces pyruvic acid.

Species Methylophilus viscogenes

A series of isolations were conducted on samples of soil and water which originated in the operating vicinity of a methanol manufacturing plant in Bishop, Texas. The samples were screened for the presence of methanol-utilizing microorganisms.

A portion of each sample was inoculated into 250 ml Erlenmeyer flasks containing methanol (0.5% v/v) and an appropriate nutrient medium, e.g., the mineral salts(MS) medium illustrated in Table I, plus one gram per liter of ammonium chloride. The flasks were incubated at various temperatures ranging from 30°-55° C. After detectable turbidity indicative of growth occurred, 2 ml samples were removed and employed to inoculate similar sterile flasks of medium. A number of subsequent subcultures were taken for each original sample, then the cultures were streaked out on medium agar plates to obtain single microorganism-derived colonies from which pure cultures of methanol-assimilating microorganisms were obtained. Strain ATCC 39893 was isolated and obtained as a biologically pure culture in this manner.

Strains of Methylophilus viscogenes exhibit a novel combination of properties that distinguish them from other methylotrophic bacteria. A Methylophilus viscogenes bacterium such as strain ATCC 39893 is a facultative methylotroph which utilizes the ribose monophosphate pathway of $C_1$ assimilation, and which typically has a growth rate doubling time of 1-3 hours at 35°-40° C.

Strain ATCC 39893 grows on methanol, fructose and glucose. In addition, it will grow on a wider variety of heterotrophic substrates (e.g., succinate and pyruvate) when an exogenous energy supply in the form of formate or methanol is present. This is a property not previously described for any known microorganism. For purposes of identification, bacteria manifesting this phenomenon are now termed "latent facultative methylotrophs".

Bacteria having the characteristics comprising those of strain ATCC 39893 are further identified by non-slimy growth of pale orange colonies on solid media. Another identifying characteristic of a strain ATCC 39893 type of methylotrophic bacteria is a hexulose phosphate synthase/hexalose phosphate isomerase activity of at least about 400 nanomoles of NADH formed per minute per milligram of protein.

A significant property of a strain ATCC 39893 type of facultative methylotroph is the ability to bioconvert a methanol substrate into an accumulated quantity of an exopolysaccharide which imparts pseudoplastic and thixotropic properties to aqueous solutions. A strain ATCC 39893 bacterium is particularly unique in its ability to produce a large quantity of accumulated exopolysaccharide in the cultivation medium in the presence or absence of cell growth and under a variety of culture conditions. A strain ATCC 39893 bacterium has the inherent ability to divert a large percentage of available carbon source to biosynthesis of an exopolysaccharide..

A further characteristic of a strain ATCC 39893 type of bacteria is a logarithmic pattern of growth, as opposed to an exponential growth phase. A strain ATCC 39893 culture exhibits a natural metabolic dysfunction which prevents unrestricted growth. When the fermentation medium is supplemented with a variety of growth factors such as vitamins, aminoacids, or yeast extract, the logarithmic growth is not affected.

A Methylophilus viscogenes strain can be cultured aerobically in a nutrient medium comprising a carbon source, nitrogen source, salts, and various growth promoters.

Suitable nitrogen sources include ammonium sulfate, ammonium chloride, ammonia, diammonium phosphate, ammonium nitrate, sodium nitrate, urea, corn steep liquor, casein, peptone, yeast extract, meat extract, and the like.

Suitable mineral salts include calcium salts, magnesium salts, potassium salts, phosphate salts, iron salts, manganese salts, zinc salts, copper salts, and the like. Bacterial growth promoters include soy bean protein hydrolysate, yeast extract, vitamins, and aminoacids.

Cultivation of the microorganism in the nutrient medium typically is conducted aerobically at a temperature of 35°-40° C and a pH between about 6.7-7.1 by means of shaken or submerged cultivation.

Employing the cultivation conditions previously described, a strain of Methylophilus viscogenes produces and accumulates an exopolysaccharide in a high concentration and with a high rate of carbon source utilization.

Strain ATCC 39893 is capable of producing the exopolysaccharide in an amount up to about 1.5 grams per liter, based on the culture medium, when methanol is used as the growth carbon source. The yield of exopolysaccharide typically will vary in the range between about 30-50 percent, based on the quantity of methanol utilized.

A detailed disclosure of novel species Methylophilus viscogenes is set forth in copending patent application Serial Number 700,562, filed February 11, 1985, incorporated in its entirety by reference herein.

The following examples are further illustrative of the present invention. The components and specific ingredients are presented as being typical, and various modifications can be derived in view of the foregoing disclosure within the scope of the invention.

For growth of methanol-assimilating bacteria a mineral salts medium(MS) (Table I) is employed. The medium is either supplemented with 1 g/L potassium nitrate, giving nitrate mineral salts medium(NMS), or 1 g/L ammonium chloride, giving ammonium mineral salts medium(AMS). The carbon source, methanol, preferably is present at a concentration between 0.2-0.5% (v/v).

For solid media, 17 g/L of Difco bacto-agar is added to the basic mineral salts medium (minus phosphates) prior to sterilization. Sterile phosphate solution is added aseptically to the sterile mineral salts medium on cooling.

Fermentation procedures are conducted in a B. Braun instruments biostat S fermentor (6 liters). A 3 liter working volume is used, and methanol is added aseptically after sterilization of the fermentor. A stirring rate of 500 rpm is routinely used with an air delivery rate of 460 mls m$^{-1}$. The fermentor is equipped with pH and dissolved oxygen control.

Hexulose phosphate synthase/hexulase phosphate isomerase are assayed simultaneously, since there is no assay for hexulose 6-phosphate which is the product of hexulose phosphate synthase activity. Hexulose phosphate isomerase converts hexulose 6-phosphate to glucose 6-phosphate which can be estimated using glucose 6-phosphate dehydrogenase and following the concomitant NADP+ reduction at 340 nm. The enzyme assay solution contains (final conc.): phosphate buffer 50 mM, pH 7.2; magnesium chloride 2.5 mM; glucose 6-phosphate dehydrogenase 0.7 units; phosphoglucoisomerase 0.75 units; phosphoribose isomerase 1.75 units; ribose 5-phosphate 5 mM; NADP+ 0.25 mM; and formaldehyde 5 mM.

Any presence of hydroxypyruvate reductase is indicative of the serine pathway of formaldehyde assimilation in the microorganism. This is determined by an enzyme assay solution which contains (final conc.): potassium phosphate, 1 M, pH 6.3; lithium hydroxy pyruvate 0.01 M; and NADH 2 mM. NADH disappearance is measured at 340 nm.

The estimation of glycerol and dihydroxyacetone is accomplished with glycerol dehydrogenase/dihydroxyacetone reductase (glycerol:NAD+ 2-oxidoreductase, EC 1.1.1.6). The enzyme is derivated from Enterobacter aerogenes, and is available from Sigma Biochemicals.

For the estimation of glycerol, the reaction mixture (1.0 ml) contains 50 mole TRIS-HCl buffer (Sigma Biochemicals), pH 9.7; 0.2 mole NAD+; 1 unit enzyme; and 15 mole glycerol (or test solution/fermentation broth, 20-50 1). Assays are initiated by addition of substrate and followed by monitoring increasing absorbance at 340 nm in a Beckman Model 25 UV spectrophotometer.

For the estimation of dihydroxyacetone, the reaction mixture (o.1 ml) contains 50 mole phosphate buffer, pH 6.0; 0.5 mole NADH; 1 unit enzyme; 15 mole dihydroxyacetone (or test solution/fermentation broth, 20-50 ml). Assays are initiated by addition of substrate and followed by monitoring decreasing absorbance at 340 nm.

The exopolysaccharide (e.g., heteropolysaccharide Poly 54) is recovered after the completion of a Methylotrophus viscogenes strain cultivation run. The broth is removed from the fermentor, and whole cells are removed by centrifugation (13,000 xg for 10 minutes). The exopolysaccharide is isolated by precipitation, and the product is subjected to alkaline treatment to produce deacetylated heteropolysaccharide. Alternative procedures for deacetylating the heteropolysaccharide are described herein.

Rheological measurements on aqueous solutions of polysaccharides are made on a Wells/Brookfield cone/plate digital viscometer or a Rheomat 30 viscometer fitted with a coaxial cylinder sensor system.

The xanthan gum employed for comparative viscosity measurements is Kelzan, which is a commercial product sold by Kelco Co., San Diego, California.

Analysis of the monosaccharide content of an exopolysaccharide is accomplished by a hydrolysis-gas liquid chromatography method.


EXAMPLE I

This Example illustrates the culturing of a Methylotrophus viscogenes strain to form an accumulated quantity of exopolysaccharide metabolite, and the subsequent alkaline treatment to form deacetylated heteropolysaccharide.

A 500 ml inoculum of strain ATCC 39893 is grown employing MS medium, one gram per liter of ammonium chloride, and 0.5% (v/v) methanol as a growth carbon source. The flask is incubated at 37°C for two days. A 14 liter New Brunswick Microferm fermentor containing 10 liters of MS medium, one gram per liter of ammonium chloride, and 0.5% (v/v) methanol is inoculated with the above culture.

Initial fermentation conditions are as follows: 37°C, agitation 200 rpm, air flow 2 liters/minute, and pH 7.0. The pH is controlled at about 7.0 during the fermentation. Methanol is added intermittenly to provide a concentration of 0.5% (v/v) whenever the fermentation medium becomes carbon exhausted. The concentration of methanol is determined by gas-liquid chromatography. After 24-36 hours of fermentation, the agitation rate is increased to 400 rpm.

The fermentation is terminated when methanol is no longer being utilized by the culture (usually 5-7 days). The highly viscous fermentation broth is then diluted with isopropanol (2:1 alcohol:broth) to facilitate precipitation of the oxopolysaccharide. The resulting slurry is blanketed with a nitrogen atmosphere.

The pH of the slurry is adjusted to pH 12 with sodium hydroxide to effect the deacetylation reaction. After a reaction period of two hours while maintaining pH at 12 by addition of more NaOH as needed, the aqueous medium is neutralized to pH 7 with hydrochloric acid, and the deacetylated exopolysaccharide (i.e., deacetylated heteropolysaccharide) precipitate is collected by filtration. The precipitate is washed with about one volume of isopropanol, then air dried or dried at 55°C in a forced air oven. The dried solids subsequently are milled to a powder in a Wiley Mill or hammer mill. The physicochemical properties of the deacetylated heteropolysaccharide product correspond to those described hereinabove for deacetylated heteropolysaccharide Poly 54.

The culture procedure described above is repeated to provide a fermentation broth containing an accumulated quantity of heteropolysaccharide Poly 54 in solution. The pH of the aqueous fermentation medium is adjusted to pH 12 with sodium hydroxide, and after two hours at pH 12 the fermentation medium is neutralized with hydrochloric acid. The deacetylated heteropolysaccharide Poly 54 product is precipitated and recovered following the procedure described above.

## TABLE I

### MS MEDIUM

| | |
|---|---|
| $MgSO_4$ $7H_2O$ | 1 g |
| $CaCl_2$ | 0.2 g |
| $Na_2HPO_4$ | 0.33 g |
| $KH_2PO_4$ | 0.26 g |
| FeEDTA | 5.0 mg |
| $CuCl_2 \cdot H_2O$ | 2.0 mg |
| $Na_2MoO_4 \cdot 2H_2O$ | 2.0 mg |
| $FeSO_4 \cdot 7H_2O$ | 500 g |
| $ZnSO_4 \cdot 7H_2O$ | 400 g |
| $MnCl_2 \cdot 4H_2O$ | 20 g |
| $H_3BO_4$ | 15 g |
| $CoCl_2 \cdot 6H_2O$ | 50 g |
| $NiCl_2 \cdot 6H_2O$ | 10 g |
| EDTA | 250 g |
| $H_2O$ | 1 liter    pH 6.8 |

EXAMPLE II

This Example illustrates a process embodiment in accordance with the present invention.

Heteropolysaccharide Poly 54 is produced and recovered as a solid product following the general procedure of Example I.

A 0.5 g quantity of Poly 54 is dissolved in 50 milliliters of water. An aqueous solution of potassium hydroxide is added to the Poly 54 solution to adjust the pH to 12, while nitrogen is being bubbled through the Poly 54 solution. The solution viscosity increases to the consistency of a soft gel.

An aqueous solution of hydrochloric acid is added to the gelled solution to neutralize the alkalinity to a pH of 6.8-7. The neutralized solution is poured into aqueous isopropanol (1:1) to precipitate deacetylated heteropolysaccharide Poly 54. The precipitate is collected by filtration, washed with 70% isopropanol, and dried in a 55°C oven. The dried product is ground to a fine powder in a mill.

Deacetylated heteropolysaccharide Poly 54 powder is dissolved in deionized water, and in 1% sodium chloride solution, to form 0.25% solutions, respectively. In a similar manner, 0.25% solutions of heteropolysaccharide Poly 54 are prepared.

The viscosities of the solutions are measured, employing a Brookfield RVT, Spindle 4, over a range of RPM speeds. The results of the comparative viscosity measurements are summarized in Table II.

The viscosity data demonstrate that deacetylated heteropolysaccharide Poly 54 produced in accordance with the present invention exhibits greater thickening power in aqueous solutions than does the corresponding acetylated heteropolysaccharide Poly 54.

Deacetylated heteropolysaccharide Poly 54 imparts pseudoplastic and thixotropic properties to aqueous solutions.

Figure 1 is a graphic representation of comparative viscosity data employing Spindle 4 for 0.25% solutions of deacetylated Poly 54, xanthan gum, and deacetylated xanthan gum. The data demonstrate the exceptional viscosity-enhancing properties of deacetylated heteropolysaccharide Poly 54 in aqueous solutions.

## TABLE II

| Solvent | Viscosity of Poly 54[1] | | Viscosity of Deacetylated Poly 54[1] | |
|---|---|---|---|---|
| | RPM | Viscosity (cps) | Rpm | Viscosity (cps) |
| Deionized Water | 0.5 | 800 | 0.5 | 13600 |
| | 1 | 1400 | 1 | 9200 |
| | 2.5 | 1000 | 2.5 | 6200 |
| | 5 | 520 | 5 | 2920 |
| | 10 | 340 | 10 | 1800 |
| | 20 | 210 | 20 | 1110 |
| | 50 | 108 | 50 | 528 |
| 1% NaCl | 0.5 | - | 0.5 | 3200 |
| | 1 | 200 | 1 | 2000 |
| | 2.5 | 200 | 2.5 | 1600 |
| | 5 | 120 | 5 | 920 |
| | 10 | 80 | 10 | 520 |
| | 20 | 70 | 20 | 350 |
| | 50 | 36 | 50 | 196 |

(1) Brookfield RVT, spindle 4; 0.25% solution.

**0 215 623**

**Claims**

1. A fermentation process which comprises aerobically cultivating a Methylophilus viscogenes strain in an aqueous nutrient medium containing a growth carbon source to produce an accumulated quantity of extracellular heteropolysaccharide in the fermentation medium, separating the culture microorganism from the fermentation medium to provide a cell-free aqueous solution of heteropolysaccharide, and adjusting the pH of the aqueous solution into the alkaline range to deacetylate the heteropolysaccharide.

2. A fermentation process in accordance with claim 1 wherein the growth carbon source is a $C_1$-compound.

3. A fermentation process in accordance with claim 1 wherein the growth carbon source is methanol.

4. A fermentation process in accordance with claim 1 wherein the growth carbon source is fructose.

5. A fermentation process in accordance with claim 1 wherein the strain is ATCC 39893.

6. A deacetylated heteropolysaccharide produced in accordance with the fermentation process of claim 1.

7. Deacetylated heteropolysaccharide Poly 54 produced in accordance with the fermentation process of claim 5.

8. A fermentation process which comprises aerobically cultivating Methylophilus viscogenes strain ATCC 39893 or a variant thereof in a nutrient medium containing methanol as a growth carbon source to produce an accumulated quantity of extracellular heteropolysaccharide Poly 54 in the fermentation medium, and reacting the heteropolysaccharide Poly 54 under alkaline conditions to produce deacetylated heteropolysaccharide Poly 54.

9. A fermentation process in accordance with claim 8 wherein the deacetylated heteropolysaccharide Poly 54 is recovered from the aqueous medium as a solid product.

10. Deacetylated heteropolysaccharide Poly 54, produced in accordance with the process of claim 8.

11. A process which comprises hydrolyzing heteropolysaccharide Poly 54 in an aqueous medium under alkaline pH conditions to produce deacetylated heteropolysaccharide Poly 54.

12. A process in accordance with claim 11 wherein the alkaline treatment is conducted at a solution pH between about 8-12 and a reaction temperature between about 25°-100°C.

13. A fermentation process which comprises aerobically cultivating a Methylophilus viscogenes strain in an aqueous nutrient medium containing a growth carbon source to produce an accumulated quantity of extracellular heteropolysaccharide in the aqueous medium, diluting the aqueous medium with a water-miscible organic solvent to precipitate the heteropolysaccharide to form an aqueous slurry medium, treating the aqeuous slurry medium with an alkaline reagent to produce an aqueous slurry of deacetylated heteropolysaccharide.

14. A fermentation process in accordance with claim 13 wherein the growth carbon source is a $C_1$-compound.

15. A fermentation process in accordance with claim 13 wherein the growth carbon source is methanol.

16. A fermentation process in accordance with claim 13 wherein the growth carbon source is fructose.

17. A fermentation process in accordance with claim 13 wherein the strain is ATCC 39893.

18. A deacetylated heteropolysaccharide produced in accordance with the fermentation process of claim 13.

19. Deacetylated heteropolysaccharide Poly 54, produced in accordance with the fermentation process of claim 17.

20. A process which comprises contacting solid heteropolysaccharide Poly 54 with an alkaline solution to produce deacetylated heteropolysaccharide Poly 54 in solid form.

21. Deacetylated heteropolysaccharide Poly 54 which exhibits a 0.5% viscosity of at least 1000 cps at 50 RPM. as measured with Brookfield Spindle No. 4 at 25°C.

9

# FIG.I
## DEACETYLATED POLY 54 VS. XANTHAN GUM

+ - POLY 54, DEACETYLATED   ◇-XANTHAN   △-XANTHAN, DEACETYLATED